# EUROPÄISCHE PATENTSCHRIFT

(11) **EP 0 668 858 B1**
(45) Veröffentlichungstag und Bekanntmachung des Hinweises auf die Patenterteilung: **08.01.1997**
(21) Anmeldenummer: 93924561.9
(22) Anmeldetag: 30.10.1993
(51) Int. Cl.: C07D 251/16, C07D 239/47, A01N 47/36

(54) **HERBIZIDE SULFONYLHARNSTOFFE, VERFAHREN ZUR HERSTELLUNG UND IHRE VERWENDUNG**
SULPHONYL UREA COMPOUNDS WITH A HERBICIDAL ACTION, METHOD OF PREPARING THEM AND THEIR USE
SULFONYLUREES A ACTION HERBICIDE, LEUR PROCEDE DE FABRICATION ET LEUR UTILISATION

(30) Priorität: 12.11.1992 DE 4238175
(43) Veröffentlichungstag der Anmeldung: 30.08.1995
(73) Patentinhaber: BASF Aktiengesellschaft, 67063 Ludwigshafen (DE)
(72) Erfinder: MAYER, Horst, D-67069 Ludwigshafen (DE); HAMPRECHT, Gerhard, D-69469 Weinheim (DE); WESTPHALEN, Karl-Otto, D-67346 Speyer (DE); GERBER, Matthias, D-67117 Limburgerhof (DE); WALTER, Helmut, D-67283 Obrigheim (DE)
(86) Internationale Anmeldenummer: EP9303038
(87) Internationale Veröffentlichungsnummer: WO9411358

(56) Entgegenhaltungen:
- EP-A- 0 048 143
- EP-A- 0 070 802
- EP-A- 0 338 424
- EP-A- 0 388 873
- EP-A- 0 476 403
- WO-A-92/09608
- US-A- 4 169 719

## Beschreibung

Die vorliegende Erfindung betrifft Sulfonylharnstoffe der allgemeinen Formel I 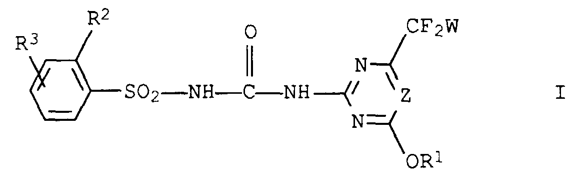 in der
- R¹: eine Methyl- oder Ethylgruppe;
- R²: C₁-C₃-Alkoxycarbonyl, eine C₁-C₂-Alkylgruppe, die 1 bis 5 Fluoratome trägt, Methylsulfonyl, Dimethylaminosulfonyl, Methylthio, Methylsulfinyl, Methylsulfonyloxy, Trifluormethoxy, Difluormethoxy, Difluorchlormethoxy, Difluorchlormethyl, Nitro;
- R³: Wasserstoff, Methyl, Methoxy, Ethoxy, Fluor, Chlor, Methylthio;
- W: Wasserstoff oder Chlor und
- Z: CH oder N
bedeuten, sowie deren landwirtschaftlich brauchbare Salze.

In der US-A 4 127 405 ist als nächstliegende Struktur die Triazinverbindung A 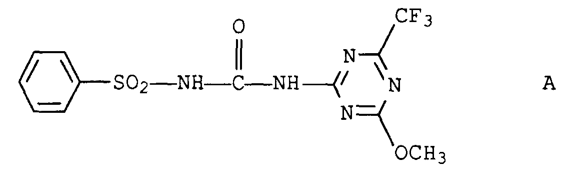 sowie in der US-A 4 169 719 das Pyrimidin-Derivat B beschrieben.

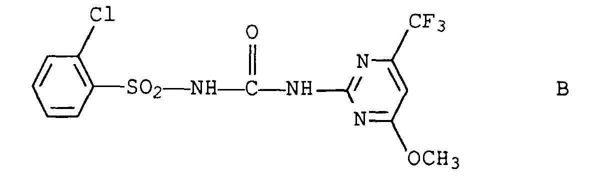

In der EP-A 44 808 sind Sulfonylharnstoffe D mit einer Substitution im Aromatenteil beschrieben. 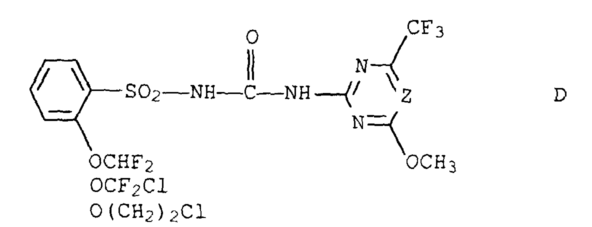 Z = CH, N

In der EP-A 48 143 sind zwei N-methylierte Sulfonylharnstoffe E ohne nähere Charakterisierung aufgeführt. 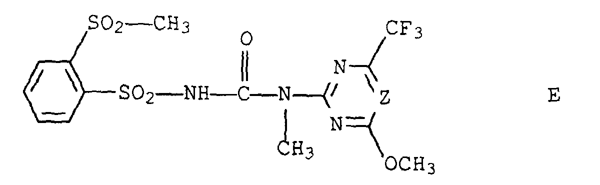 Z = CH, N

Die EP-A 388 873 umfaßt Benzoesäureester der Struktur F. 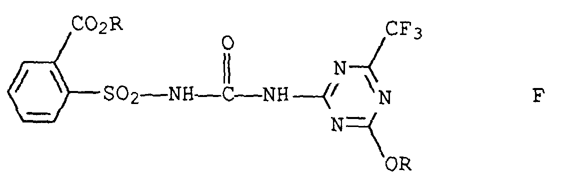 R = CH₃, C₂H₅

Im US-Patent 4 310 346 sind Sulfonamide des Typs G aufgelistet. 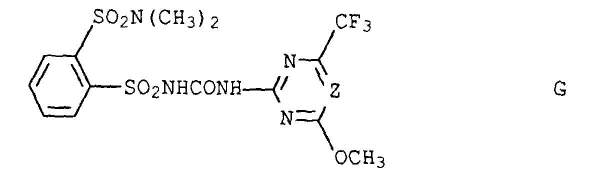 Z = CH, N

Die deutsche Offenlegungsschrift DE-OS 40 38 430 (WO 92/09608) beschreibt Trifluormethylsubstituierte Triazine des Typs H. 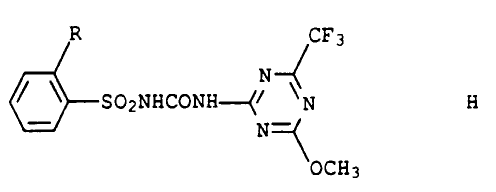
- R =: Halogen, CF₃ Alkylsulfonyl oder O(CH₂)₂OCH₃

Die EP-A 120 814 führt ohne Angabe von physikalischen Daten die Verbindung J auf.

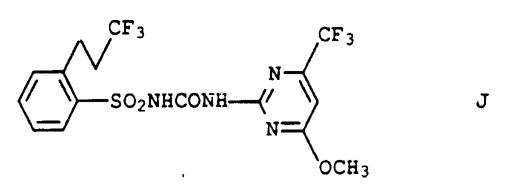

Der Erfindung lag nun die Aufgabe zugrunde, Sulfonylharnstoffe zu synthetisieren, die gegenüber den bekannten Vertretern dieser Herbizid-Klasse verbesserte Eigenschaften aufweisen und sich insbesondere durch hohe Selektivität in empfindlichen Kulturen auszeichnen.

Entsprechend dieser Aufgabe wurden die eingangs definierten Sulfonylharnstoffe der Formel I gefunden.

In der Formel I bedeutet C₁-C₃-Alkoxycarbonyl, Methoxycarbonyl, Ethoxycarbonyl, n-Propoxycarbonyl oder iso-Propoxycarbonyl und eine C₁-C₂-Alkylgruppe, die 1 bis 5 Fluoratome trägt, Methyl, substituiert durch 1 bis 3 Fluoratome oder Ethyl, substituiert durch 1 bis 5 Fluoratome z.B. Trifluormethyl, Difluormethyl, 2,2,2-Trifluorethyl oder 1,1,2,2-Tetrafluormethyl.

Besonders bevorzugt sind Sulfonylharnstoffe der Formel I, in der R² Methoxycarbonyl, Trifluormethyl, Dimethylaminosulfonyl, Trifluormethoxy, Difluormethoxy und Methylsulfonyl bedeutet. Ferner Sulfonylharnstoffe mit einem Triazinsubstituenten (Z-N). Von besonderer Bedeutung sind weiterhin Verbindungen I mit Difluormethylsubstitution aus Heteroatomen (W=H).

Die erfindungsgemäßen Sulfonylharnstoffe der Formel I sind auf verschiedenen Wegen zugänglich, die in der Literatur beschrieben sind. Beispielhaft seien besonders vorteilhafte Wege (A-C) im folgenden näher erläutert. 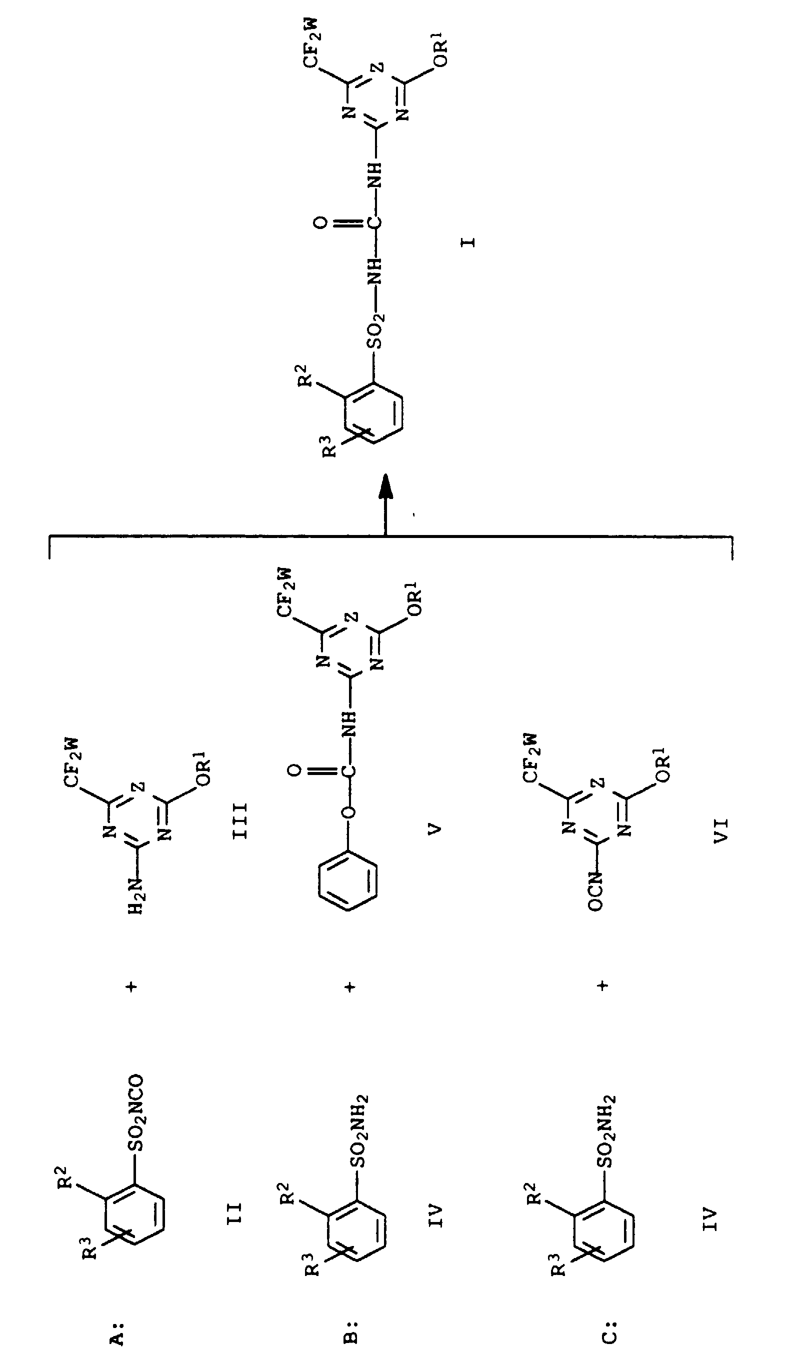
A: Man setzt ein Sulfonylisocyanat II in an sich bekannter Weise (EP-A-162 723) mit ungefähr der stöchiometrischen Menge eines 2-Amino-1,3,5-triazin- bzw. 2-Aminopyrimidin-Derivats III bei einer Temperatur von 9 bis 120°C, vorzugsweise 10 bis 100°C um. Die Reaktion kann unter Normaldruck oder unter Druck (bis 50 bar), vorzugsweise bei 1 bis 5 bar, kontinuierlich durchgeführt werden.
   Zweckmäßigerweise verwendet man für die Umsetzungen unter den jeweiligen Reaktionsbedingungen inerte Lösungs- und Verdünnungsmittel. Als Lösungsmittel kommen beispielsweise Halogenkohlenwasserstoffe, insbesondere Chlorkohlenwasserstoffe, z.B. Tetrachlorethylen, 1,1,2,2- oder 1,1,2,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trifluorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzcl, Jodbenzol, o-, m-, p-Dichlorbenzol, o-, p-, m-Dibrombenzol, o-, m-, p-Chlortoluol, 1,2,4-Trifluorbenzol; Ether, z.B. Ethylpropylether, Methyl-tert.-butylether, n-Butylethylester, Di-n-butylether, Diisobutylether, Diisoamylether, Diisopropylether, Diisopropylether, Anisol, Phenetol, Cyclohexylmethylether, Diethylether, Ethylenglykoldimethylether, Tetrahydrofuran, Dioxan, Thioanisol, β,β'-Dichlordiethylether, Nitrokohlenwasserstoffe wie Nitromethan, Nitroethan, Nitrobenzol, o-, m-, p-Chlornitrobenzol, o-Nitrotoluol; Nitrile wie Acetonitril, Butyronitril, Isobutyronitril, Benzonitril, m-Chlorbenzonitril; aliphatische oder cycloaliphatische Kohlenwasserstoffe, z.B. Heptan, Pinan, Nonan, o-, m-, p-Cymol, Benzinfraktionen innerhalb eines Siedepunktintervalles von 70 bis 190°C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan; Ester, z.B. Ethylacetat, Acetessigester, Isobutylacetat; Amide, z.B. Formamid, Methylformamid, Dimethylformamid; Ketone, z.B. Aceton, Methylethylketon und entsprechende Gemische in Betracht. Zweckmäßig verwendet man das Lösungsmittel in einer Menge von 100 bis 2000 Gew.-%, vorzugsweise von 200 bis 700 Gew.-%, bezogen auf den Ausgangsstoff II.
   Die zur Umsetzung benötigte Verbindung II wird im allgemeinen in etwa äquimolaren Mengen (mit einem Über- oder Unterschuß von z.B. 0 bis 20 %, bezogen auf den jeweiligen Ausgangsstoff III) eingesetzt. Man kann den Ausgangsstoff III in einem der vorgenannten Verdünnungsmittel vorlegen und dann den Ausgangsstoff II zugeben.
   Zweckmäßigerweise wird das Verfahren zur Herstellung der neuen Verbindungen jedoch so durchgeführt, daß man den Ausgangsstoff II, gegebenenfalls in einem der vorgenannten Verdünnungsmittel, vorlegt und dann den Ausgangsstoff III zugibt.
   Zur Beendigung der Umsetzung rührt man nach der Zugabe der Komponenten noch 20 Minuten bis 24 Stunden bei 0 bis 120°C, vorzugsweise 10 bis 100°C, nach.
   Als Reaktionsbeschleuniger kann man vorteilhafterweise ein tertiäres Amin, z.B. Pyridin, α,β,γ-Picolin, 2,4-, 2,6-Lutidin, 2,4, 6-Collidin, p-Dimethylaminopyridin, Trimethylamin, Triethylamin, Tri(n-propyl)amin, 1,4-Diaza[2,2,2]bicyclooctan [DABCO] oder 1,8-Diazabicylco[5,4,0]-undec-7-en in einer Menge von 0.01 bis 1 Mol pro Mol Ausgangsstoff II verwenden.
   Aus dem Reaktionsgemisch wird der Endstoff I in üblicher Weise, z.B. nach Abdestillieren von Lösungsmittel oder direkt durch Absaugen isoliert. Der verbleibende Rückstand kann noch mit Wasser bzw. verdünnter Säure zur Entfernung basischer Verunreinigungen gewaschen werden. Man kann jedoch auch den Rückstand in einem mit Wasser nicht mischbaren Lösungsmittel lösen und wie beschrieben waschen. Die gewünschten Endstoffe fallen hierbei in reiner Form an, gegebenenfalls können sie durch Umkristallisation, Rühren in einem organischen Lösungsmittel, das die Verunreinigungen aufnimmt oder Chromatographie gereinigt werden.
   Bevorzugt führt man diese Umsetzung in Acetonitril, Methyltert.-butylether, Toluol oder Methylenchlorid in Anwesenheit von 0 bis 100 Moläquivalenten, vorzugsweise 0 bis 50 Moläquivalenten eines tertiären Amins wie 1,4-Diazabicyclo[2,2,2]octan oder Triethylamin durch.
B: Man setzt ein Sulfonamid der Formel IV in an sich bekannter Weise (EP-A 141 777 und EP-A 101 670) in einem inerten organischen Lösungsmittel mit ungefähr der stöchiometrischen Menge eines Phenylcarbamats V bei einer Temperatur von 0 bis 120°C, vorzugsweise 20 bis 100°C um. Dic Reaktion kann bei Normaldruck oder unter Druck (bis 50 bar), vorzugsweise bei 1 bis 5 bar, kontinuierlich oder diskontinuierlich durchgeführt werden.
   Es können hierbei Basen wie tertiäre Amine zugesetzt werden, die die Reaktion beschleunigen und die Produktqualität verbessern. Geeignete Basen sind hierfür die unter A angegebenen, insbesondere Triethylamin, 2,4,6-Collidin, 1,4-Diazabicyclo[2,2,2]octan [DABCO] oder 1,8-Diazabicyclo[5,4,0]undec-7-en (DBU), in einer Menge von 0,01 bis 1 mol pro Mol Ausgangsstoff IV.
   Zweckmäßigerweise verwendet man als Löse- oder Verdünnungsmittel die unter A angegebenen.
   Man verwendet das Lösungsmittel in einer Menge von 100 bis 2000 Gew.-% vorzugsweise von 200 bis 700 Gew.-%, bezogen auf das Edukt IV.
   Die zur Umsetzung benötigte Verbindung IV wird im allgemeinen in etwa äquimolaren Mengen (mit einem Über- oder Unterschuß von z.B. 0 bis 20 %, bezogen auf die jeweiligen Ausgangsstoffe V) eingesetzt. Man kann den Ausgangsstoff V in einem der vorgenannten Verdünnungsmittel vorlegen und dann den Ausgangsstoff IV zugeben.
   Man kann jedoch auch den Ausgangsstoff IV in einem der genannten Lösungsmittel vorlegen und dann das Carbamat V zugeben. In beiden Fällen kann als Katalysator vor oder während der Reaktion eine der genannten Basen zugesetzt werden.
   Zur Beendigung der Umsetzung rührt man nach der Zugabe der Komponenten noch 20 Minuten bis 24 Stunden bei 0 bis 120°C, vorzugsweise 10 bis 100°C, insbesondere 20 bis 80°C, nach.
   Man isoliert die Sulfonylharnstoffe der Formel I aus dem Reaktionsgemisch mit den üblichen Methoden, wie unter A beschrieben.
C: Man setzt ein Sulfonamid der Formel IV in an sich bekannter Weise (EP-A 234 352) in einem inerten organischen Lösungsmittel mit ungefähr der stöchiometrischen Menge eines Isocyanats VI zu einer Temperatur von 0 bis 150°C, vorzugsweise 10 bis 100°C um. Die Reaktion kann bei Normaldruck oder unter Druck (bis 50 bar), vorzugsweise bei 1 bis 5 bar, kontinuierlich oder diskontinuierlich durchgeführt werden.
   Es können hierbei vor oder während der Reaktion Basen wie tertiäre Amine zugesetzt werden, die die Reaktionsbeschleuniger und die Produktqualität verbessern. Geeignete Basen sind hierfür die unter A angegebenen, insbesondere Triethylamin oder 2,4,6-Collidin, in einer Menge von 0,01 bis 1 mol pro Ausgangsstoff IV.
   Zweckmäßigerweise verwendet man als Lösungsmittel die unter A angegebenen. Man setzt das Lösungsmittel in einer Menge von 100 bis 2000 Gew.-%, vorzugsweise von 200 bis 700 Gew.-%, bezogen auf das Edukt IV.
   Die zur Umsetzung benötigte Verbindung IV wird im allgemeinen in etwa äquimolaren Mengen (mit einem Über- oder Unterschuß von z.B. 0 bis 20 %, bezogen auf die Edukte VI) eingesetzt. Man kann den Ausgangsstoff VI in einem der genannten Verdünnungsmittel vorlegen und dann den Ausgangsstoff IV zugeben. Man kann aber auch das Sulfonamid vorlegen und dann das Isocyanat VI zugeben.
   Zur Beendigung der Umsetzung rührt man nach der Zugabe der Komponenten noch 20 Minuten bis 24 Stunden bei 0 bis 120°C, vorzugsweise 10 bis 100°C, insbesondere 20 bis 80°C, nach. Das Endprodukt I kann aus dem Reaktionsgemisch in der üblichen Weise, wie unter A: beschreiben, gewonnen werden.

Die als Ausgangsstoffe benötigten Sulfonylisocyanate der Formel II lassen sich in an sich bekannter Weise aus den entsprechenden Sulfonamiden durch Phosgenierung (Houben-Weyl 11/2 (1985) 1106, US 4 379 769) oder durch Umsetzung der Sulfonamide mit Chlorsulfonylisocyanat (DE-A 31 32 944) gewinnen.

Carbamate der Formel V sind nach oder in Analogie zu bekannten Umsetzungen (z.B. EP-A 101 670) zugänglich, sie lassen sich aber auch aus den entsprechenden Isocyanaten VI durch Umsetzung mit Phenol herstellen.

Die Isocyanate der Formel VI erhält man aus den Aminen der Formel III durch Behandlung mit Oxalylchlorid oder Phosgen (in Analogie nach Angew. Chem. 83 (1971) 407, EP-A 388 873).

Die Sulfonamide lassen sich durch Reaktion der entsprechenden Sulfonsäurechloride mit Ammoniak gewinnen (Houben-Weyl, Methoden der organischen Chemie, Band 9 (1955) 605). Die Sulfonsäurechloride erhält man durch Meerwein-Reaktion (Diazotierung geeigneter Amine und Kupfersalz-katalysierte Sulfochlorierung).

2-Amino-4-chlordifluormethyl-6-methoxy-1,3,5-triazin und 2-Amino-4-difluormethyl-6-methoxy-1,3,5-triazin lassen sich, wie im Herstellbeispiel illustriert, synthetisieren. Analog lassen sich die entsprechenden 6-Ethoxy-substituierten 1,3,5-Triazine herstellen.

Die entsprechenden Pyrimidine der allgemeinen Formel III sind durch folaende Sequenz zugänglich:

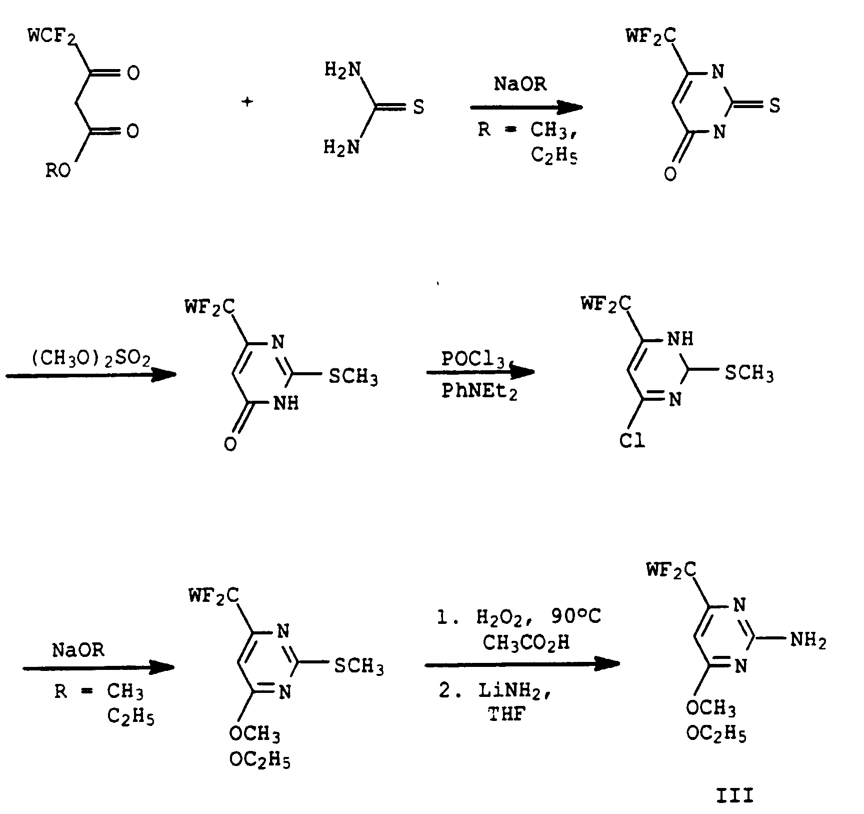

Entsprechende Umsetzungen sind gut bekannt (D.J. Brown in "The Chemistry of Heterocyclic Compounds", Interscience Publishers, New York, London, Bd. 14., Heterocycl. Chem. 20 (1983) 219).

Die Verbindungen I können in Form ihrer landwirtschaftlich brauchbaren Salze vorliegen, wobei es auf die Art des Salzes im allgemeinen nicht ankommt. Üblicherweise werden die Salze von solchen Basen in Betracht kommen, welche die herbizide Wirkung von I nicht negativ beeinträchtigen.

Die Salze der Verbindungen I sind in an sich bekannter Weise zugänglich (EP-A-304 282, US-A 4,599,412). Man erhält sie durch Deprotonierung der entsprechenden Sulfonylharnstoffe I in Wasser oder einem inerten organischen Lösungsmittel bei Temperaturen von -80°C bis 120°C, vorzugsweise 0°C bis 60°C in Gegenwart einer Base.

Geeignete Basen sind beispielsweise Alkali- oder Erdalkalimetallhydroxide, -hydride, -oxide oder -alkoholate wie Natrium-, Kalium- und Lithiumhydroxid, Natriummethanolat, -ethanolat und -tert.-butanolat, Natrium- und Calziumhydrid und Calziumoxid. Ferner können Salze von Übergangsmetallen, vorzugsweise Mangan-, Kupfer-, Zink- und Eisensalze sowie die Ammoniumsalze, die ein bis drei C₁-C₄-Alkyl-, Hydroxy-C₁-C₄-alkylsubstituenten und/oder einen Phenyl- oder Benzylsubstituenten tragen können, vorzugsweise Diisopropylammonium-, Tetramethylanmonium-, Tetrabutylammonium-, Trimethylbenzylammonium- und Trimethyl-12-hydroxyethyl)-ammoniumsalze, die Phosphoniumsalze, die Sulfoniumsalze, vorzugsweise Tri-(C₁-C₄)-alkylsulfoniumsalze, und die Sulfoxoniumsalze, vorzugsweise Tri-(C₁-C₄)-alkylsulfoxoniumsalze als basische Salze eingesetzt werden.

Als Lösungsmittel kommen beispielsweise neben Wasser auch Alkohole wie Methanol, Ethanol and tert.-Butanol, Ether wie Tetrahydrofuran und Dioxan, Acetonitril, Dimethylformamid, Ketone wie Aceton und Methylethylketon und auch halogenierte Kohlenwasserstoffe in Betracht.

Die Deprotonierung kann bei Normaldruck oder bei Drucken bis 50 bar, vorzugsweise bei Normaldruck bis 5 bar Überdruck durchgeführt werden.

Die Verbindungen I bzw. die sie enthaltenden herbiziden Mittel sowie deren umweltverträgliche Salze von Alkalimetallen und Erdalkalimetallen können in Kulturen wie Weizen, Reis und Mais Schadpflanzen sehr gut bekämpfen, ohne die Kulturpflanzen zu schädigen, ein Effekt, der vor allem auch bei niedrigen Aufwandmengen auftritt. Sie können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen u.a. Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier-oder Emulgiermittel in Wasser homogenisiert werden. Es können aber auch aus wirksamer Substanz, Netz-, Baft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkyl-arylpolyetheralkohole, Isotridecylalkohol, Fettalkoholethylenoxid-Kondensate, ethoxyliertes Rizinusöl, Polyoxyethylenalkylether oder Polyoxypropylenalkylether Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,1 und 95 Gew.-%, vorzugsweise zwischen 0,5 und 90 Gew.-%, Wirkstoff. Die Wirkstoffe werden dabei in einer Reinheit von 90 bis 100 %, vorzugsweise 95 bis 100 % (auch NMR-Spectrum) eingesetzt.

Beispiele für solche Zubereitungen sind:
I 20 Gew.-Teile der Verbindung Nr. 1.01 werden in einer Mischung gelöst, die aus 80 Gew.-Teilen alkyliertem Benzol, 10 Gew.-Teilen des Anlagerungsproduktes von 8 bis 10 Mol Ethylenoxid an 1 Mol Ölsäure-N-monoethanolamid, 5 Gew.-Teilen des Anlagerungsproduktes von 40 Mol Ethylenoxid an 1 Mol Ricinusöl besteht. Durch Ausgießen und feines Verteilen der Lösung in 100 000 Gew.-Teilen Wasser erhält man eine wäßrige Dispersion, die 0,02 Gew.-% des Wirkstoffs enthält.
II 20 Gew.-Teile der Verbindung Nr. 1.01 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
III 20 Gew.-Teile des Wirkstoffs Nr. 1.01 werden in einer Mischung gelöst, die aus 25 Gew.-Teilen Cyclohexanon, 65 Gew.-Teilen einer Mineralölfraktion vom Siedepunkt 210 bis 280°C. und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
IV 20 Gew.-Teile des Wirkstoffs Nr. 1.01 werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser enthält man eine Spritzbrühe, die 0,1 Gew.-% des Wirkstoffs enthält.
V 3 Gew.-Teile des Wirkstoffs Nr. 1.01 werden mit 97 Gew.-Teilen feinteiligem Kaolin vermischt. Man erhält auf diese Weise ein Stäubemittel, das 3 Gew.-% des Wirkstoffs enthält.
VI 20 Gew.-Teile des Wirkstoffs Nr. 1.01 werden mit 2 Gew.-Teilen Calciumsalz der Dodecylbenzolsulfonsäure, 8 Gew.-Teilen Fettalkohol-polyglykolether, 2 Gew.-Teilen Natriumsalz eines Phenol-Harnstoff-Formaldehyd-Kondensates und 68 Gew.-Teilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 1,0, vorzugsweise 0,01 bis 0,5 kg/ha aktive Substanz (a.S.).

In Anbetracht der Vielseitigkeit der Applikationsmethoden können die Sulfonylharnstoffe I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis Guineensis, Fragaria vesca, Glycine max, Gossypium hirsutum (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicaco sativa, Musa spp., Nicotiana tabacum (N. rustica), Olea europaea, Oryza sativa, Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (S. vulgare), Theobroma cacao, Trifolium pratense, Triticum aestivum, Triticum durum, Vicia faba, Vitis vinifera und Zea mays.

Zur Verbreiterung des Wirkungsspektrums und zur Erzielung synergistischer Effekte können die substituierten Sulfonylharnstoffe der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Phenyloxy- bzw. Heteroaryloxy-phenylpropionsäuren sowie deren Salze, Ester und Amide und andere in Betracht.

Außerdem kann es von Nutzen sein, die Verbindungen der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

Nachstehend sind Beispiele für die Synthese der Verbindungen I wiedergegeben.

### Herstellung der Ausgangsstoffe

### 2-Amino-4-chlordifluormethyl-6-methoxy-1,3,5-triazin

a) 2-Amino-4-chlordifluormethyl-6-thiomethyl-1,3,5-triazin
   Eine Suspension von 18,5 g N-Amidinothioharnstoff (Addukt mit N-Methyl-2-pyrrolidon) (85 mmol) in 90 ml Methanol wurde mit 10,7 g Dimethylsulfat (85 mmol) versetzt, 3 h bei 30 bis 40°C gerührt. Zu dieser Lösung tropfte man bei 0°C 25 g Chlordifluoressigsäuremethylester (0,17 mol) gefolgt von 30,6 g einer 30-gewichtsprozentigen Lösung von Natriummethanolat in Methanol (0,17 mol). Man entfernte die Kühlung und rührte 16 h bei 25°C nach. Das Lösungsmittel wurde bei 40°C entfernt, der Rückstand mit 400 ml Wasser gerührt, das Produkt abgesaugt und im Wasserstrahlvakuum bei 40°C getrocknet. Das Rohprodukt (Fp. 118°C) wurde ohne Reinigung in Stufe b) eingesetzt (¹H-NMR-Spektrum (250 MHz, CDCl₃, int. TMS, δ (ppm): 6,74 br (1H): 5,94 br (1H): 2,54 s (3H)).
b) 2-Amino-4-chlordifluormethyl-6-methoxy-1,3,5-triazin
   Eine Lösung von 19 g Rohprodukt (84 mmol) aus Stufe a) in 100 ml Methanol wurde bei 0°C tropfenweise mit 16,2 g einer 30 gew.-%igen Lösung von Natriummethanolat in Methanol (90 mmol) versetzt. Man entfernte die Kühlung und rührte 16 h bei 25°C nach. Durch Zugabe von 4N HCl wurde ein pH-Wert von 6 eingestellt, die Lösungsmittel bei 40°C im Wasserstrahlvakuum entfernt und der Rückstand mit 400 ml Wasser gerührt. Das Produkt wurde abgesaugt, mit Wasser gewaschen und im Wasserstrahlvakuum bei 40°C getrocknet.
   Man erhielt so 13,9 g der Titelverbindung (79 % d. Th.) mit Fp. 130°C (¹H-NMR-Spektrum (250 MHz, CDCl₃, int. TMS, δ (ppm): 6,78 br (1H): 6,08 br (1H): 4,03 s (3H)).

### 2-Amino-4-difluormethyl-6-methoxy-1,3,5-triazin

a) 2-Amino-4-difluormethyl-6-trichlormethyl-1,3,5-triazin
   Eine Lösung von 70,1 g Difluoressigsäureanhydrid (0,4 mol) in 200 ml Diethylether wurde portionsweise mit 40,7 g N-(Trichloracetamidino)guanidin (0,2 mol) bei 0°C versetzt. Der Ansatz wurde 3 Stunden bei 20 bis 25°C gerührt. Die flüchtigen Anteile wurden bei 40°C im Wasserstrahlvakuum entfernt, der Rückstand zwischen 400 ml Wasser und 200 ml Methylenchlorid verteilt und die Methylenchloridphase vorsichtig mit verdünnter Natronlauge (2 gew.-%ig) neutralisiert. Nach Abtrennen und Trocknen der Methylenchloridphase über Na₂SO₄ wurde das Lösungsmittel im Wasserstrahlvakuum bei 40°C abdestilliert. Man erhält so 39,6 g (0,15 mol) (75 % d. Th.) eines spektroskopisch reines Rohprodukt, das so in die Folgeumsetzung (Stufe b) ohne Reinigung eingesetzt werden kann.
   ¹H-NMR-Spektrum (270 MHz, d₆-DMSO, int. TMS, δ (ppm): 8,80 br (2H): 6,78 tr (1H;J_{H-F} 162 Hz)).
b) 2-Amino-4-difluormethyl-6-methoxy-1,3,5-triazin
   Eine Lösung von 22,5 Rohprodukt (85 mmol) aus Stufe a) in 100 ml Methanol wurde bei 0°C tropfenweise mit 1,6 g einer 30 gew.-%igen Lösung von Natriummethanolat in Methanol (9 mmol) versetzt. Man entfernte die Künlung und rührte 16 Stunden bei 25°C nach. Nach Zugabe von weiteren 1,6 g einer 30 gew.-%igen Lösung von Natriummethanolat in Methanol (9 mmol) wurde 3 Stunden bei 25°C nachgerührt. Durch Zugabe von 3 N Salzsäure wurde ein pH-Wert von 7 eingestellt, die Lösungsmittel bei 40°C im Wasserstrahlvakuum entfernt und der Rückstand kräftig mit 400 ml Wasser gerührt. Das Produkt wurde abgesaugt, mit Wasser gewaschen und im Wasserstrahlvakuum bei 40°C getrocknet.
   Man erhielt so 10,7 g der Titelverbindung (0,61 mol; 71 % d. Th.). ¹H-NMR-Spektrum (270 MHz, d₆-DMSO, int. TMS, δ (ppm): 8,02, 7,94 br (2H); 6,55 tr (1H;J_{H-F} 162 Hz); 3,90 s (3H)).

### Herstellung der Sulfonylharnstoffe der Formel I

### Beispiel 1.03

### [2-[[(4-Chlordifluormethyl-6-methoxy-1,3,5-triazin-2-yl)aminocarbonyl]aminosulfonyl]benzoesäuremethylester

Eine Lösung von 4,2 g 2-Amino-4-chlordifluormethyl-6-methoxy-1,3,5-triazin (20 mmol) in 20 ml Methylenchlorid wurde bei 25°C mit einer Lösung von 4,8 g 2-(Methoxycarbonyl)benzolsulfonylisocyanat (20 mmol) in 5 ml Methylenchlorid versetzt. Man rührte 16 h bei 25°C nach, entfernte das Lösungsmittel im Wasserstrahlvakuum bei 40°C und rührte den festen Rückstand mit 100 ml einer Hexan/Diethylether-Mischung (v:v, 1:1). Das abgeschiedene Produkt wurde abgesaugt, mit wenig Ether gewaschen und getrocknet. Durch Umkristallisieren aus Methanol/Wasser erhielt man die Titelverbindung (3,2 g, 35 % d.Th.) mit Fp. 174 bis 175°C.

### Beispiel 1.05

N-[(4-Chlordifluormethyl-6-methoxy-1,3,5-triazin-2-yl)aminocarbonyl]-2-nitrobenzolsulfonamid

Eine Lösung von 4,2 g 2-Amino-4-chlordifluormethyl-6-methoxy-1,3,5-triazin (20 mmol) in 20 ml Methylenchlorid wurde bei 25°C mit einer Lösung von 4,6 g 2-Nitrobenzolsulfonylisocyanat (20 mmol) in 5 ml Methylenchlorid versetzt. Man rührte 16 h bei 25°C nach, saugte das abgeschiedene Produkt ab, wusch mit wenig Ether und trocknete im Wasserstrahlvakuum bei 40°C. Man erhielt so 3,1 g der Titelverbindung (35 % d.Th.) mit Fp. 181°C.

### Beispiel 1.06

### Natrium[N-[(4-Chlordifluormethyl-6-methoxy-1,3,5-triazin-2-yl)aminocarbonyl]-2-nitrobenzolsulfonamid]

Eine Suspension von 1,5 g N-[(4-Chlordifluormethyl-6-methoxy-1,3,5-triazin-2-yl)aminocarbonyl]-2-nitrobenzolsulfonamid (3,4 mmol) in 10 ml Methanol wurde mit 0,62 g einer 30 gew.-%igen Lösung von Natriummethanolat (3,4 mmol) in Methanol bei 25°C versetzt, worauf Lösung eintrat. Man rührte 30 min bei 25°C nach, entfernte die flüchtigen Anteile bei 40°C im Wasserstrahlvakuum. Man erhielt so die Titelverbindung in quantitativer Ausbeute mit einem Zersetzungspunkt von 169°C.

### Beispiel 3.01

### 2-[[(4-Difluormethyl-6-methoxy-1,3,5-triazin-2-yl)aminocarbonyl]aminosulfonyl]benzotrifluorid

Eine Lösung von 2,65 g 2-Amino-4-difluormethyl-6-methoxy-1,3,5-triazin (15 mmol) in 20 ml Acetonitril wurde bei 25°C mit 3,7 g 2-(Trifluormethyl)benzolsulfonylisocyanat (15 mmol) versetzt. Man rührte 16 h bei 25°C nach, entfernte das Lösungsmittel im Wasserstrahlvakuum bei 40°C und rührte den festen Rückstand kräftig mit 100 ml einer Diethylether. Das Produkt wurde abgesaugt, mit wenig Ether gewaschen und getrocknet. Man erhielt 4,3 g (10 mmol, 67 % d. Th.) der Titelverbindung mit Fp. 143-145°C.

### Beispiel 3.02

### Natrium[N- [(4-difluormethyl-6-methoxy-1,3,5-triazin-2-yl)-aminocarbonyl]aminosulfonylbenzotrifluorid]

Eine Suspension von 1,3 g 2-[[(4-Difluormethyl-6-methoxy-1,3,5-triazin-2-yl)aminocarbonyl]aminosulfonyl]benztrifluorid (3 mmol) in 10 ml Methanol wurde mit 0,54 g einer 30 gew.-%igen Lösung von Natriummethanolat (3 mmol) in Methanol bei 25°C versetzt, worauf Lösung eintrat. Man rührte 30 min bei 25°C nach, entfernte die flüchtigen Anteile bei 40°C im Wasserstrahlvakuum. Man erhielt so die Titelverbindung in quantitativer Ausbeute mit einem Zersetzungspunkt von 189-192°C.

Die in den nachfolgenden Tabelle 1 bis 4 genannten Wirkstoffe werden auf analogem Herstellungsweg erhalten.

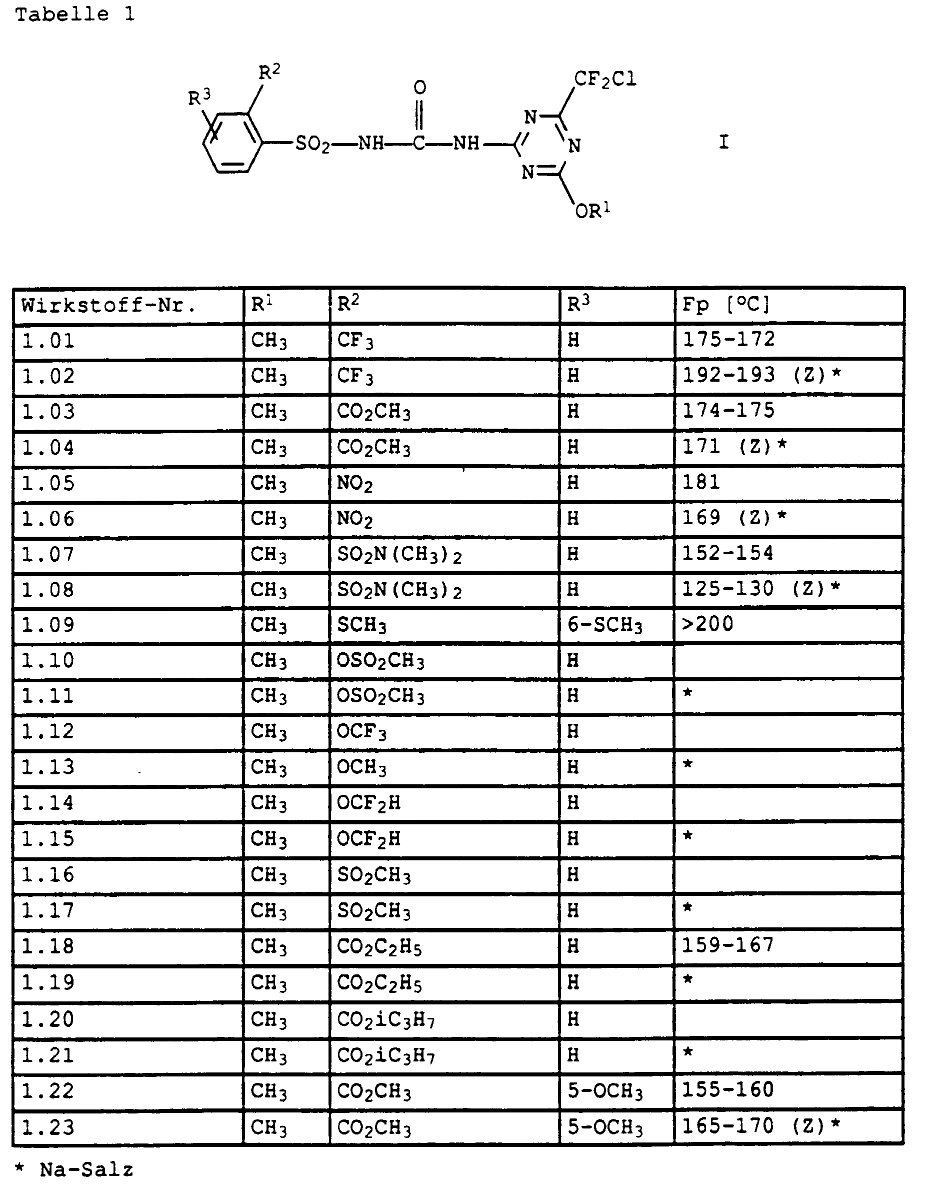

In analoger Weise können auch die nachfolgenden aufgeführten Verbindungen erhalten werden: 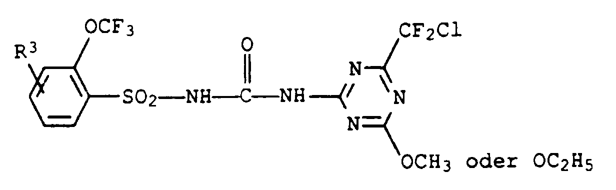 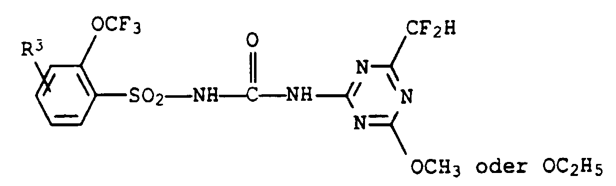 oder deren Salze Na-Salze, wobei R³ die folgende Bedeutung hat:
3-Methyl, 5-Methyl, 6-Methyl, 3-Methylthio, 5-Methylthio, 6-Methylthio, 3-Fluor, 5-Fluor, 6-Fluor, 3-Chlor, 5-Chlor, 6-Chlor, 3-Methoxy, 4-Methoxy, 5-Methoxy, 6-Methoxy, 3-Ethoxy, 5-Ethoxy, 6-Ethoxy; 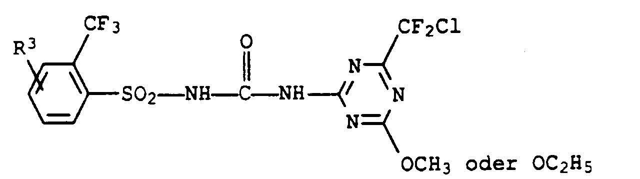 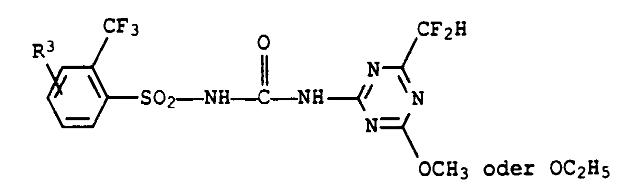 oder deren Salze Na-Salze, wobei R³ die folgende Bedeutung hat:
3-Methyl, 5-Methyl, 6-Methyl, 3-Methylthio, 5-Methylthio, 6-Methylthio, 3-Fluor, 5-Fluor, 6-Fluor, 3-Chlor, 5-Chlor, 6-Chlor, 3-Methoxy, 4-Methoxy, 5-Methoxy, 6-Methoxy, 3-Ethoxy, 5-Ethoxy, 6-Ethoxy; 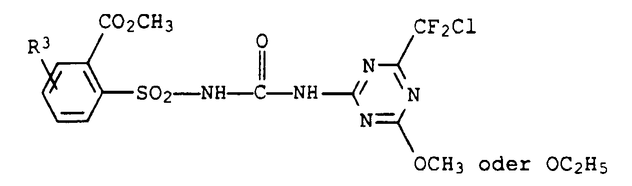 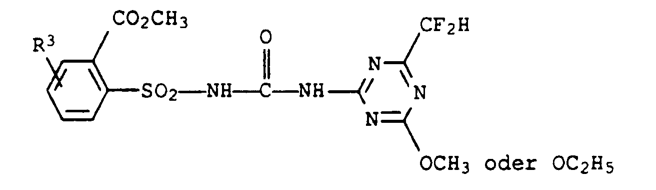 oder deren Salze Na-Salze, wobei R³ die folgende Bedeutung hat:
3-Methyl, 5-Methyl, 6-Methyl, 3-Methylthio, 5-Methylthio, 6-Methylthio, 3-Fluor, 5-Fluor, 6-Fluor, 3-Chlor, 5-Chlor, 6-Chlor, 3-Methoxy, 4-Methoxy, 5-Methoxy, 6-Methoxy, 3-Ethoxy, 5-Ethoxy, 6-Ethoxy; 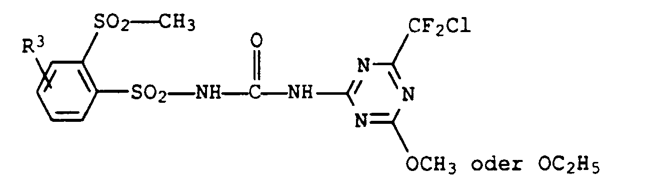 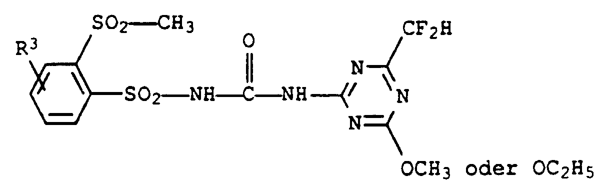 oder deren Salze Na-Salze, wobei R³ die folgende Bedeutung hat:
3-Methyl, 5-Methyl, 6-Methyl, 3-Methylthio, 5-Metylthio, 6-Methylthio, 3-Fluor, 5-Fluor, 6-Fluor, 3-Chlor, 5-Chlor, 6-Chlor, 3-Methoxy, 4-Methoxy, 5-Methoxy, 6-Methoxy, 3-Ethoxy, 5-Ethoxy, 6-Ethoxy; 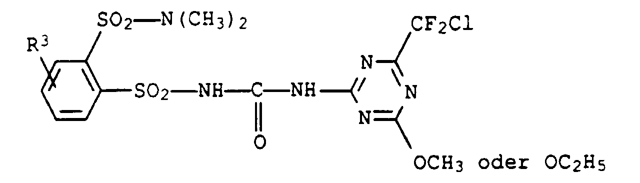 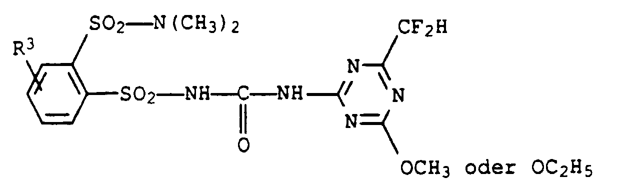 oder deren Salze Na-Salze, wobei R³ die folgende Bedeutung hat :
3-Methyl, 5-Methyl, 6-Methyl, 3-Methylthio, 5-Methylthio, 6-Methylthio, 3-Fluor, 5-Fluor, 6-Fluor, 3-Chlor, 5-Chlor, 6-Chlor, 3-Methoxy, 4-Methoxy, 5-Methoxy, 6-Methoxy, 3-Ethoxy, 5-Ethoxy, 6-Ethoxy; 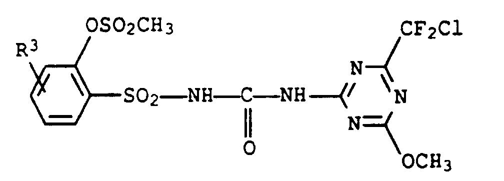 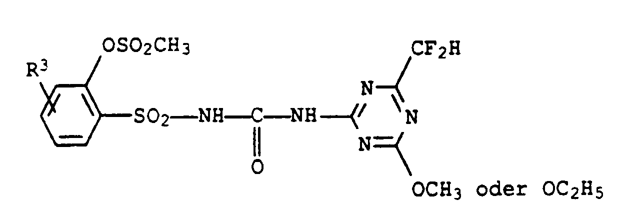 oder deren Salze Na-Salze, wobei R³ die folgende Bedeutung hat:
3-Methyl, 5-Methyl, 6-Methyl, 3-Methylthio, 5-Methylthio, 6-Methylthio, 3-Fluor, 5-Fluor, 6-Fluor, 3-Chlor, 5-Chlor, 6-Chlor, 3-Methoxy, 4-Methoxy, 5-Methoxy, 6-Methoxy, 3-Ethoxy, 5-Ethoxy, 6-Ethoxy; 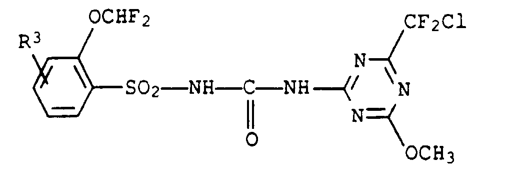 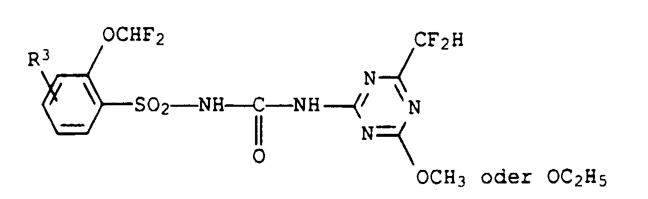 oder deren Salze Na-Salze, wobei R³ die folgende Bedeutung hat:
3-Methyl, 5-Methyl, 6-Methyl, 3-Methylthio, 5-Methylthio, 6-Methylthio, 3-Fluor, 5-Fluor, 6-Fluor, 3-Chlor, 5-Chlor, 6-Chlor, 3-Methoxy, 4-Methoxy, 5-Methoxy, 6-Methoxy, 3-Ethoxy, 5-Ethoxy, 6-Ethoxy.

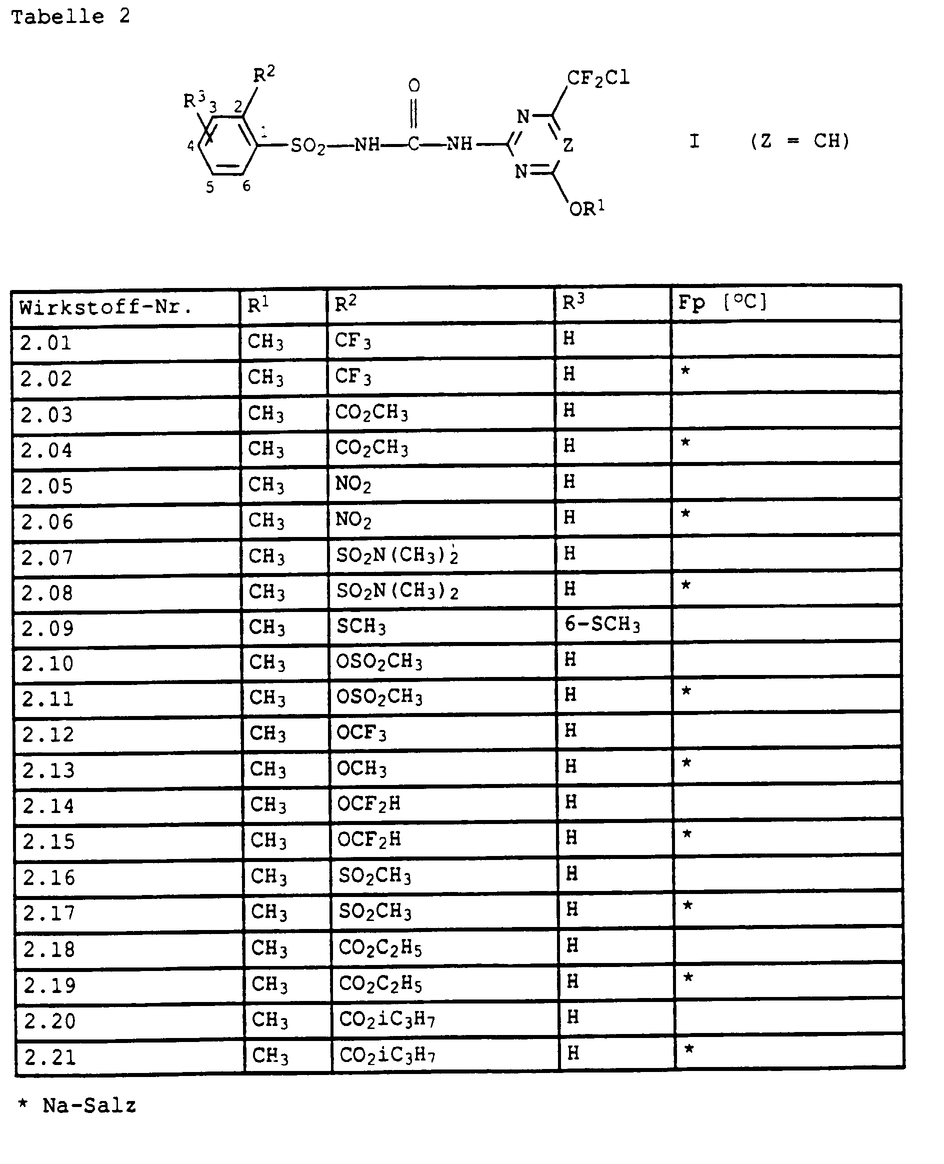

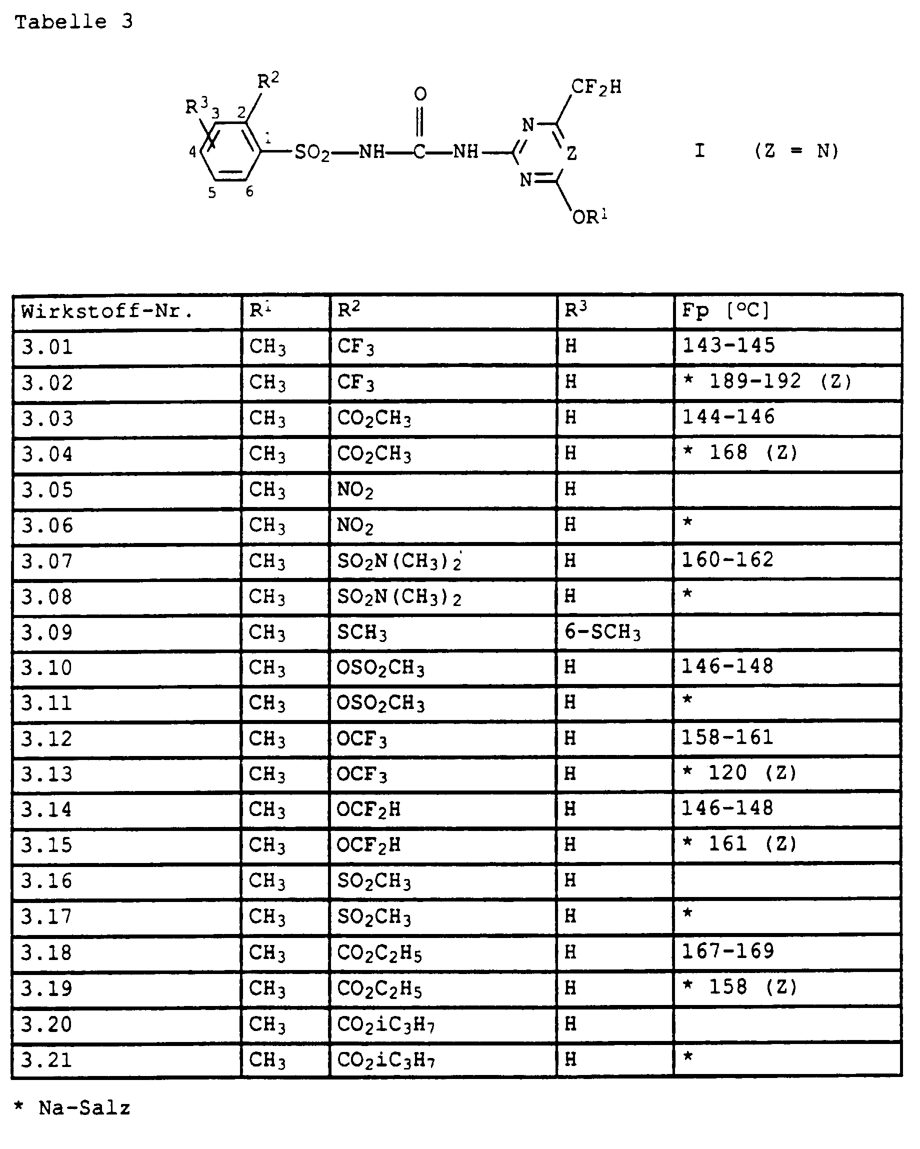

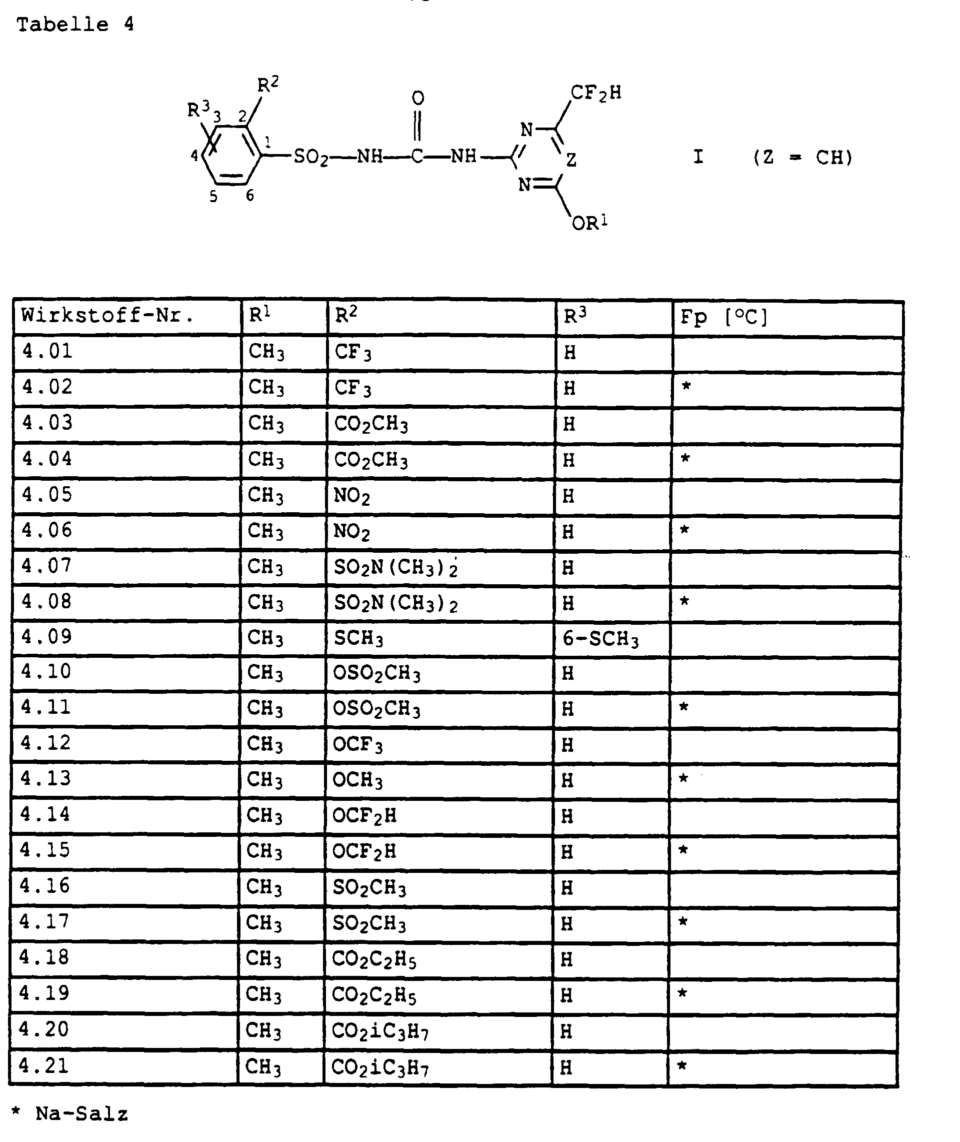

### Anwendungsbeispiele:

Die herbizide Wirkung der Sulfonylharnstoffe der Formel I auf das Wachstum der Testpflanzen wird durch folgende Gewächshausversuche gezeigt.

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefaße wurden leicht beregnet, um Keimung und Wachstum zu fordern, und anschließend mit durchsichtigen Plastikhauben abgedeckt, bis die Pflanzen angewachsen waren. Diese Abdeckung bewirkt ein gleichmlßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 4 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachaüflaufbehandlung betrug 0,06 kg/ha a.S. (aktive Substanz).

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 keine Keimung der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name |
|---|---|
| Abutilon theophrasti | Chinesischer Hanf |
| Sinapis alba | Weißer Senf |
| Zea mays | Mais |

Mit 0,06 kg/ha a.S. im Nachauflaufverfahren eingesetzt lassen sich mit Beispiel 1.03 breitblättrige unerwünschte Pflanzen sehr gut bekämpfen, bei gleichzeitiger hervorragender Selektivität in der Beispielkulturpflanze Mais.

In den folgenden Tabellen sind Ergebnisse aus biologischen Untersuchungen zusammengestellt, in denen der erfindungsgemäße Wirkstoff Beispiel 1.03 mit der aus US-A 4,169,719 bekannten Verbindung B und der erfindungsgemäße Wirkstoff Nr. 3.01 mit der aus WO 92/09608 bekannten Vergleichsverbindung H 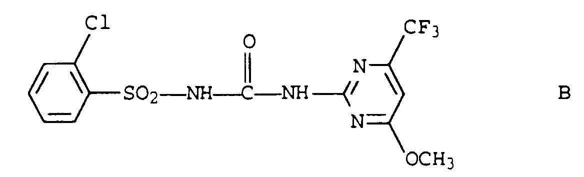 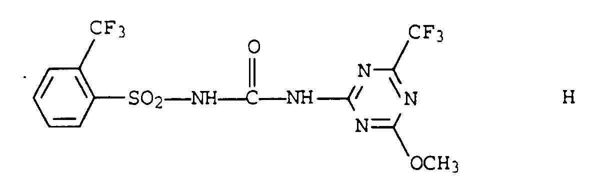 verglichen wurden.

**Tabelle I:**

| Vergleich der herbiziden Aktivität der Beispielverbindung Nr. 1.03 mit der bekannten Vergleichsverbindung B bei Nachauflaufanwendung von 0,0313 bzw. 0,0156 kg/ha a.S. im Gewächshaus. | | | | |
|---|---|---|---|---|
| Testpflanzen | Schädigung [%] Aufwandmenge [kg/ha a.S.] | | | |
| | Beispiel 1.03 | | B | |
| | 0,0313 | 0,0156 | 0,0313 | 0,0156 |
| Amaranthus retroflexus | 100 | 100 | 75 | 70 |
| Galium aparine | 95 | 75 | 85 | 40 |
| Polygonum persicaria | 80 | 80 | 40 | 40 |
| Sinapis alba | 90 | 90 | 75 | 75 |
| Solanum nigrum | 90 | 90 | 60 | 60 |
| Veronica spp. | 85 | 75 | 50 | 40 |

**Tabelle II:**

| Vergleich der herbiziden Aktivität der Beispielverbindung Nr. 3.01 mit der bekannten Vergleichsverbindung H bei Nachauflaufanwendung von 0,063 bzw. 0,0313 kg/ha a.S. im Gewächshaus. | | | | |
|---|---|---|---|---|
| Testpflanzen | Schädigung [%] Aufwandmenge [kg/ha a.S.] | | | |
| | Beispiel 3.01 | | H | |
| | 0,063 | 0,0313 | 0,063 | 0,0313 |
| Alopecurus myosuroides | 98 | 98 | 40 | 0 |
| Galium aparine | 95 | 95 | 95 | 95 |
| Ipomea spp. | 98 | 90 | 80 | 80 |
| Sinapis alba | 98 | 98 | 100 | 100 |
| | | | | |
| Triticum aestivum | 0 | 0 | 0 | 0 |

## Patentansprüche

1. Sulfonylharnstoffe der allgemeinen Formel I 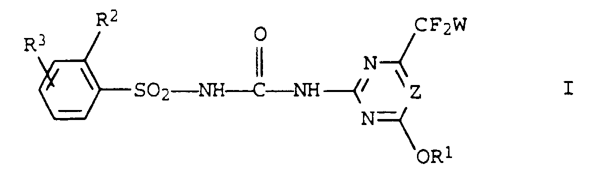 in der
R¹ eine Methyl- oder Ethylgruppe;
R² C₁-C₃-Alkoxycarbonyl, eine C₁-C₂-Alkylgruppe, die 1 bis 5 Fluoratome trägt, Methylsulfonyl, Dimethylaminosulfonyl, Methylthio, Methylsulfinyl, Methylsulfonyloxy, Trifluormethoxy, Difluormethoxy, Difluorchlormethoxy, Difluorchlormethyl, Nitro;
R³ Wasserstoff, Methyl, Methoxy, Ethoxy, Fluor, Chlor, Methylthio;,
W Wasserstoff oder Chlor und
Z CH oder N
bedeuten, sowie deren landwirtschaftlich brauchbare Salze.

2. Sulfonylharnstoffe der Formel I gemäß Anspruch 1, in der R² Methoxycarbonyl, Trifluormethyl, Dimethylaminosulfonyl, Trifluormethoxy, Difluormethoxy und Methylsulfonyl bedeutet.

3. Sulfonylharnstoffe der Formel I gemäß Anspruch 1, in der Z für Stickstoff steht.

4. Sulfonylharnstoffe der Formel I gemäß Anspruch 1, in der W für Wasserstoff steht.

5. Verfahren zur Herstellung der Sulfonylharnstoffe der Formel I gemäß Anspruch 1, dadurch gekennzeichnet, daß man ein Sulfonylisocyanat II 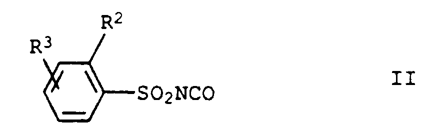 in an sich bekannter Weise in einem inerten organischen Lösungsmittel mit der ungefähr stöchiometrischen Menge eines 2-Amino-1,3,5-triazin- bzw. 2-Aminopyrimidin-Derivats III 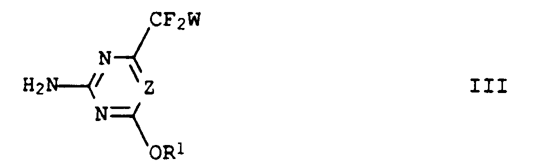 umsetzt.

6. Verfahren zur Herstellung der Sulfonylharnstoffe der Formel I gemäß Anspruch 1, dadurch gekennzeichnet, daß man ein entsprechendes Sulfonamid der Formel IV 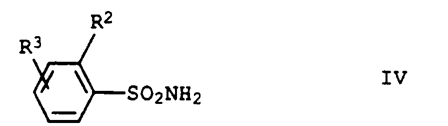 in an sich bekannter Weise in einem inerten organischen Lösungsmittel mit einem Phenylcarbamat V 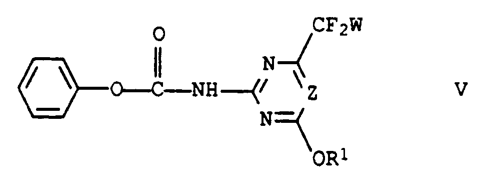 umsetzt.

7. Verfahren zur Herstellung der Sulfonylharnstoffe der Formel I gemäß Anspruch 1, dadurch gekennzeichnet, daß man ein entsprechendes Sulfonamid der Formel IV 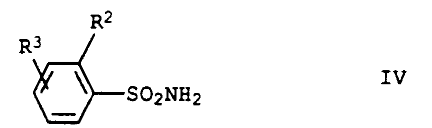 in an sich bekannter Weise in einem inerten organischen Lösungsmittel mit einem Isocyanat der Formel VI 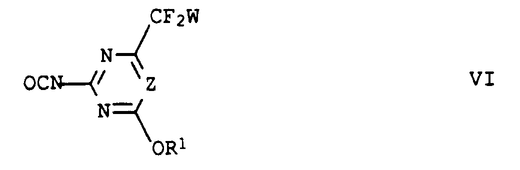 umsetzt.

8. Verfahren zur Herstellung von Salzen der Verbindungen I gemäß Anspruch 1, dadurch gekennzeichnet, daß man eine Verbindung der Formel I gemäß Anspruch 1 in an sich bekannter Weise in Wasser oder einem inerten organischen Lösungsmittel mit einer Base deprotoniert.

9. Herbizides Mittel, enthaltend einen Sulfonylharnstoff der Formel I gemäß Anspruch 1 oder sein Salz sowie hierfür übliche Trägerstoffe.

10. Verfahren zur Bekämpfung unerwünschten Pflanzenwuchses, dadurch gekennzeichnet, daß man eine herbizid wirksame Menge eines Sulfonylharnstoffs der Formel I gemäß Anspruch 1 oder eines seiner Salze auf die Pflanzen und/oder ihren Lebensraum einwirken läßt.

11. 2-Amino-1,3,5-triazine der Formel IIIa 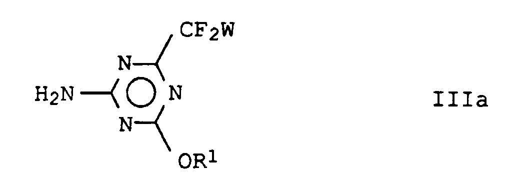 in der W Wasserstoff oder Chlor und R¹ Methyl oder Ethyl bedeuten.

## Claims

1. A sulfonylurea of the general formula I 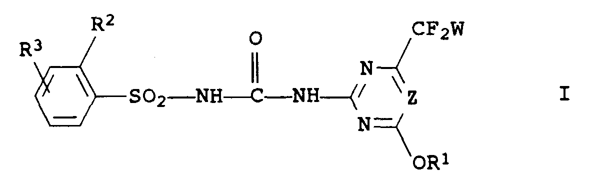 where
R¹ is a methyl or ethyl group;
R² is C₁-C₃-alkoxycarbonyl, a C₁-C₂-alkyl group which carries 1 to 5 fluorine atoms, methylsulfonyl, dimethylaminosulfonyl, thiomethyl, methylsulfinyl, methylsulfonyloxy, trifluoromethoxy, difluoromethoxy, difluorochloromethoxy, difluorochloromethyl or nitro;
R³ is hydrogen, methyl, methoxy, ethoxy, fluorine, chlorine or thiomethyl;
W is hydrogen or chlorine and
Z is CH or N
and their agriculturally utilizable salts.

2. A sulfonylurea of the formula I is claimed in claim 1, where R² is methoxycarbonyl, trifluoromethyl, dimethylaminosulfonyl, trifluoromethoxy, difluoromethoxy or methylsulfonyl.

3. A sulfonylurea of the formula I as claimed in claim 1, where Z is nitrogen.

4. A sulfonylurea of the formula I as claimed in claim 1, where W is hydrogen.

5. A process for preparing the sulfonylureas of the formula I as claimed in claim 1, which comprises reacting a sulfonyl isocyanate II 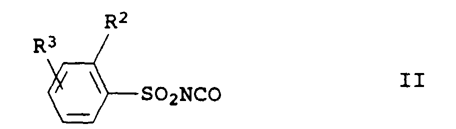 in a manner known per se in an inert organic solvent with approximately the stoichiometric amount of a 2-amino-1,3,5-triazine or 2-aminopyrimidine derivative III

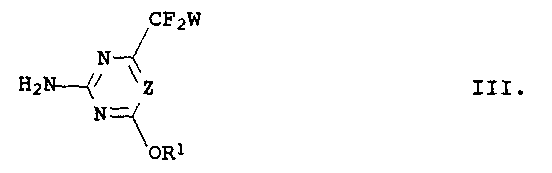

6. A process for preparing the sulfonylureas of the formula I as claimed in claim 1, which comprises reacting an appropriate sulfonamide of the formula IV 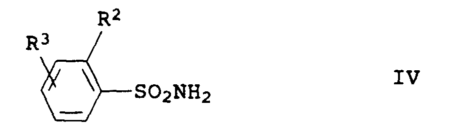 in a manner known per se in an inert organic solvent with a phenyl carbamate V

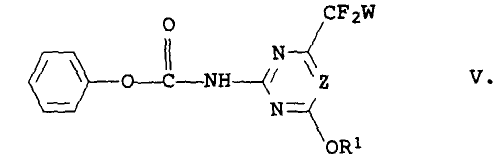

7. A process for preparing the sulfonylureas of the formula I as claimed in claim 1, which comprises reacting an appropriate sulfonamide of the formula IV 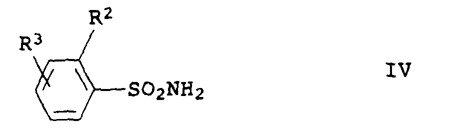 in a manner known per se in an inert organic solvent with an isocyanate of the formula VI

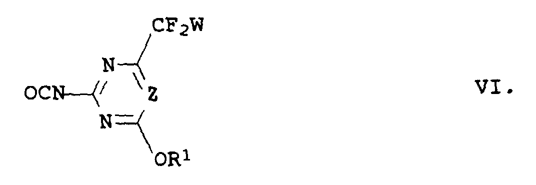

8. A process for preparing salts of the compounds I as claimed in claim 1, which comprises deprotonating a compound of the formula I as claimed in claim 1 in a manner known per se in water or an inert organic solvent using a base.

9. A herbicidal composition containing a sulfonylurea of the formula I as claimed in claim 1 or its salt and carriers customary for this purpose.

10. A process for controlling undesired plant growth, which comprises allowing a herbicidally active amount of a sulfonylurea of the formula I as claimed in claim 1 or of one of its salts to act on the plants and/or their environment.

11. A 2-amino-1,3,5-triazine of the formula IIIa 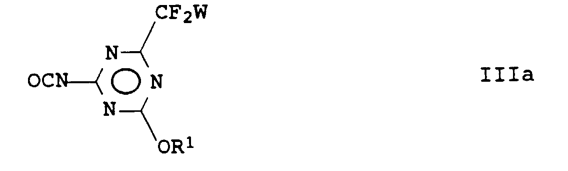 where W is hydrogen or chlorine and R¹ is methyl or ethyl.

## Revendications

1. Sulfonylurées de formule générale I 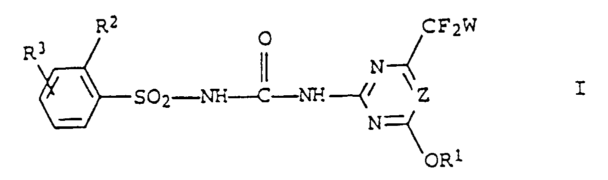 dans laquelle
R¹ représente un groupe méthyle ou éthyle ;
R² représente un groupe (alcoxy en C1-C3)carbonyle, un groupe alkyle en C1-C2 portant un à cinq atomes de fluor, un groupe méthylsulfonyle, diméthylaminosulfonyle, méthylthio, méthylsulfinyle, méthylsulfonyloxy, trifluorométhoxy, difluorométhoxy, difluorochlorométhoxy, difluorochlorométhyle, nitro ;
R³ représente l'hydrogène, un groupe méthyle, méthoxy, éthoxy, le fluor, le chlore, un groupe méthylthio ;
W représente l'hydrogène ou le chlore et
Z représente CH ou N,
et leurs sels aptes à des applications dans l'agriculture.

2. Sulfonylurées de formule I de la revendication 1 dans laquelle R² représente un groupe méthoxycarbonyle, trifluorométhyle, diméthylaminosulfonyle, trifluorométhoxy, difluorométhoxy ou méthylsulfonyle.

3. Sulfonylurées de formule I selon la revendication 1, dans laquelle Z représente l'azote.

4. Sulfonylurées de formule I de la revendication 1, dans laquelle W représente l'hydrogène.

5. Procédé de préparation des sulfonylurées de formule I de la revendication 1, caractérisé en ce que l'on fait réagir un sulfonylisocyanate II 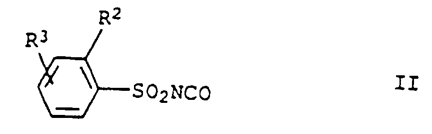 de manière connue en soi, dans un solvant organique inerte, avec une quantité voisine de la quantité théorique d'un dérivé de 2-amino-1,3,5-triazine ou de 2-aminopyrimidine III

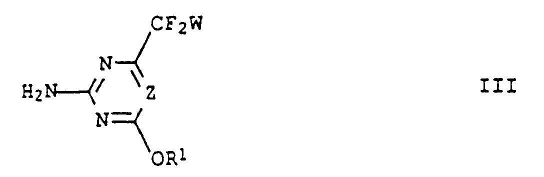

6. Procédé de préparation des sulfonylurées de formule I de la revendication 1, caractérisé par le fait que l'on fait réagir un sulfonamide correspondant de formule IV 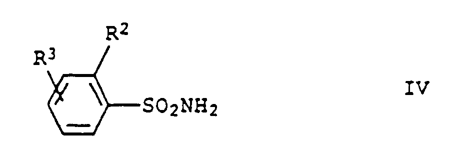 de manière connue en soi, dans un solvant organique inerte, avec un phénylcarbamate V

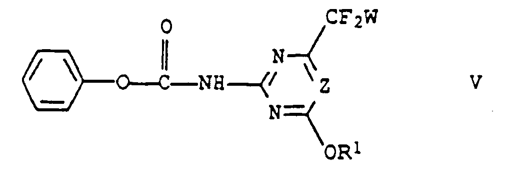

7. Procédé de préparation des sulfonylurées de formule I de la revendication 1, caractérisé par le fait que l'on fait réagir un sulfonamide correspondant de formule IV 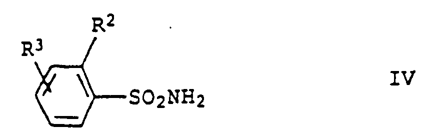 de manière connue en soi, dans un solvant organique inerte, avec un isocyanate de formule VI

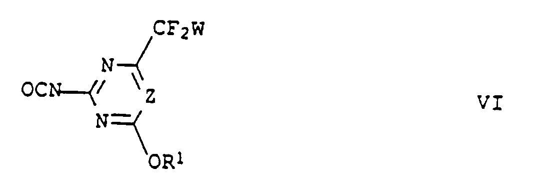

8. Procédé de préparation des sels des composés I de la revendication 1, caractérisé par le fait que l'on soumet un composé de formule I de la revendication 1 à déprotonisation à l'aide d'une base de manière connue en soi, dans l'eau ou dans un solvant organique inerte.

9. Produit herbicide contenant une sulfonylurée de formule I de la revendication 1, ou l'un de ses sels, avec des véhicules usuels à cet effet.

10. Procédé pour combattre les croissances de végétaux indésirables, caractérisé par le fait que l'on fait agir une quantité herbicide efficace d'une sulfonylurée de formule I de la revendication 1 ou de l'un de ses sels sur les végétaux et/ou leur habitat.

11. 2-amino-1,3,5-triazines de formule IIIa 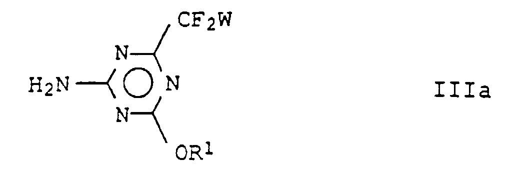 dans laquelle W représente l'hydrogène ou le chlore et R¹ un groupe méthyle ou éthyle.
